# EUROPEAN PATENT APPLICATION

(11) **EP 4 046 997 A1**
(43) Date of publication of application: **24.08.2022**
(21) Application number: 20876960.4
(22) Date of filing: 13.10.2020
(51) Int. Cl.: C07D 413/10, A01N 43/80, A01P 13/00

(54) **ISOXAZOLINE COMPOUND HAVING OPTICAL ACTIVITY AND USE THEREOF**

(30) Priority: 18.10.2019 CN 201910995340
(71) Applicant: Shenyang Sinochem Agrochemicals R & D Co., Ltd., Shenyang, Liaoning 110021 (CN); Jiangsu Yangnong Chemical Co., Ltd., Yangzhou, Jiangsu 225009 (CN)
(72) Inventor: YANG, Jichun, Shenyang, Liaoning 110021 (CN); GUAN, Aiying, Shenyang, Liaoning 110021 (CN); CUI, Dongliang, Shenyang, Liaoning 110021 (CN); WU, Qiao, Shenyang, Liaoning 110021 (CN); MA, Hongjuan, Shenyang, Liaoning 110021 (CN); WU, Enming, Shenyang, Liaoning 110021 (CN); LIU, Changling, Shenyang, Liaoning 110021 (CN)
(74) Representative: Nederlandsch Octrooibureau
(86) International application number: PCT/CN2020/120547
(87) International publication number: WO 2021/073487

(57) **Abstract**

The present invention relates to the field of agricultural herbicides, and particularly relates to an isoxazoline compound with optical activity and the uses thereof. The isoxazoline compound with optical activity is shown in the formula (I). Substituents are described in the description. The compound of the formula (I) has excellent herbicidal activity, can be used for weeding pre- and post- emergence, and can also be used for soil treatment. The compound of the formula (I) can effectively control various broad-leaved weeds, grass weeds and Cyperaceae weeds, and can control various resistant weeds, such as glyphosate-resistant weeds. The excellent weeding effect can be obtained at a low dose. The compound has good safety to crops such as corn, wheat, rice, soybean and sugar beet. The compound can be used as a selective herbicide in crop fields in agriculture, and can also be used as a non-selective herbicide in non-cultivated land, fallow land, woodland, orchards and ridges.

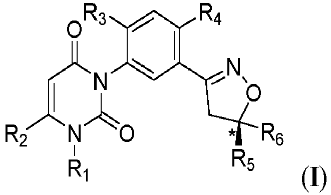

## Description

### Technical Field

The present invention belongs to the field of agricultural herbicides, and particularly relates to an isoxazoline compound with optical activity and use thereof.

### Background

CN105753853 has disclosed an isoxazoline compound having the following structural formula and herbicidal activity. However, it does not involve the report of an optical isomer of the compound, does not describe whether the herbicidal activity of optical isomers is the same or different, and even does not involve which optical isomer is more effective as the herbicidical active ingredient. It is difficult to predict the difference without specific experimental studies.

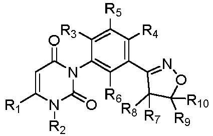

### Summary

The purpose of the present invention is to provide an isoxazoline compound with optical activity and use thereof.

To achieve the above purpose, the present invention adopts the following technical solution:

The isoxazoline compound with optical activity, which has excellent herbicidal activity, in the present invention is shown in formula (I):

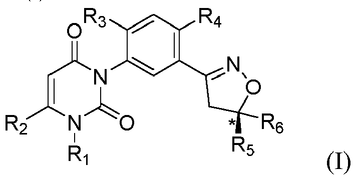

Wherein:
^{∗} represents an asymmetric carbon atom with configuration S;
R₁ is selected from CH₃ or NH₂;
R₂ is selected from C₁-C₄ alkyl or C₁-C₄ haloalkyl;
R₃ and R₄ are respectively selected from hydrogen, halogen, C₁-C₆ alkyl or C₁-C₆ alkoxy;
R₅ is selected from hydrogen, cyano, C₁-C₆ alkyl, C₁-C₆ haloalkyl, C₃-C₆ cycloalkyl, phenyl, or phenyl substituted independently with 1-4 following substitutents: halogen, CN, NO₂, C₁-C₆ alkyl, C₁-C₆ haloalkyl, C₁-C₆ alkoxy, C₁-C₆ haloalkoxy, C₁-C₆ alkylthio or C₁-C₆ alkylsulfonyl;
R₆ is selected from C₁-C₆ alkyl CO₂R₇, CO₂R₇ or CONR₈R₉;
R₇ is selected from hydrogen, C₁-C₆ alkyl, C₁-C₆ haloalkyl, C₃-C₆ cycloalkyl, C₃-C₆ alkenyl, C₃-C₆ alkynyl, C₁-C₆ alkoxy C₁-C₆ alkyl, C₁-C₆ alkoxycarbonyl C₁-C₄ alkyl, C₁-C₆ alkylcarbonyloxy C₂-C₄ alkyl, and benzyl, furylidene, thiazomethylene, tetrahydrofuranmethylene or pyridinemethylene which is unsubstituted or further substituted with 1-4 groups independently selected from the following: halogen, CN, NO₂, C₁-C₈ alkyl, C₁-C₈ haloalkyl, C₁-C₈ alkoxy, C₁-C₈ haloalkoxy, C₁-C₈ alkoxycarbonyl, C₁-C₈ alkylthio or C₁-C₈ alkylsulfonyl;
R₈ and R₉ are respectively selected from hydrogen, C₁-C₆ alkyl, C₁-C₆ haloalkyl or C₁-C₄ alkoxycarbonyl C₁-C₄ alkyl.

The configuration at a central asymmetric carbon atom represented by ^{∗} is S configuration.

A preferred compound in the present invention is: in the formula (I)
^{∗} represents an asymmetric carbon atom with configuration S;
R₁ is selected from CH₃ or NH₂;
R₂ is selected from methyl, isopropyl, tert-butyl, trifluoromethyl, trichloromethyl, difluoromethyl or heptafluoroisopropyl;
R₃ and R₄ are respectively selected from hydrogen, fluorine, chlorine, bromine, methyl, ethyl, isopropyl, tert-butyl, methoxy, ethoxy or isopropoxy;
R₅ is selected from hydrogen, cyano, C₁-C₄ alkyl, C₁-C₄ haloalkyl, C₃-C₆ cycloalkyl, phenyl, or phenyl substituted independently with 1-4 following substitutents: halogen, CN , NO₂, C₁-C₄ alkyl, C₁-C₄ haloalkyl, C₁-C₄ alkoxy, C₁-C₄ haloalkoxy, C₁-C₄ alkylthio or C₁-C₄ alkylsulfonyl;
R₆ is selected from C₁-C₄ alkyl CO₂R₇, CO₂R₇ or CONR₈R₉;
R₇ is selected from hydrogen, C₁-C₆ alkyl, C₁-C₆ haloalkyl, C₃-C₆ cycloalkyl, C₃-C₆ alkenyl, C₃-C₆ alkynyl, C₁-C₄ alkoxy C₁-C₄ alkyl, C₁-C₄ alkoxycarbonyl C₁-C₄ alkyl, C₁-C₄ alkylcarbonyloxy C₂-C₄ alkyl, and benzyl, furylmethylene or tetrahydrofurylidene which is unsubstituted or further substituted with 1-4 groups independently selected from the following: halogen, CN, NO₂, C₁-C₆ alkyl or C₁-C₆ haloalkyl;
R₈ and R₉ are respectively selected from hydrogen, C₁-C₄ alkyl, C₁-C₄ haloalkyl or C₁-C₄ alkoxycarbonyl C₁-C₄ alkyl.

A further preferred compound in the present invention is: in the formula (I)
^{∗} represents an asymmetric carbon atom with configuration S;
R₁ is selected from CH₃ or NH₂;
R₂ is selected from methyl, trifluoromethyl, trichloromethyl or difluoromethyl;
R₃ and R₄ are respectively selected from hydrogen, fluorine, chlorine, bromine, methyl, methoxy, ethoxy or isopropoxy;
R₅ is selected from hydrogen, cyano, C₁-C₄ alkyl, C₁-C₄ haloalkyl, C₃-C₆ cycloalkyl, phenyl or phenyl substituted independently with 1-4 following substitutents: halogen, CN, NO₂, C₁-C₄ alkyl, C₁-C₄ haloalkyl, C₁-C₄ alkoxy or C₁-C₄ haloalkoxy;
R₆ is selected from C₁-C₄ alkyl CO₂R₇, CO₂R₇ or CONR₈R₉;
R₇ is selected from hydrogen, C₁-C₄ alkyl, C₁-C₄ haloalkyl, C₃-C₆ cycloalkyl, C₃-C₆ alkenyl, C₃-C₆ alkynyl, C₁-C₄ alkoxy C₁-C₂ alkyl, C₁-C₄ alkoxycarbonyl C₁-C₂ alkyl, C₁-C₄ alkylcarbonyloxy C₂-C₄ alkyl, and benzyl, furylmethylene or tetrahydrofurylidene which is unsubstituted or further substituted with 1-3 groups independently selected from the following: halogen, CN, NO₂, C₁-C₄ alkyl or C₁-C₄ haloalkyl;
R₈ and R₉ are respectively selected from hydrogen, C₁-C₄ alkyl, C₁-C₄ haloalkyl or C₁-C₄ alkoxycarbonyl C₁-C₂ alkyl.

A more preferred compound in the present invention is: in the formula (I)
^{∗} represents an asymmetric carbon atom with configuration S;
R₁ is selected from CH₃ or NH₂;
R₂ is selected from trifluoromethyl, trichloromethyl or difluoromethyl;
R₃ and R₄ are respectively selected from hydrogen, fluorine, chlorine, bromine or isopropoxy;
R₅ is selected from hydrogen, cyano, methyl, ethyl, n-propyl, isopropyl, n-butyl, isobutyl, tert-butyl, trifluoromethyl, difluoromethyl, phenyl, or phenyl substituted independently with 1-4 following substitutents: halogen, CN, NO₂, methyl, isopropyl, tert-butyl, trifluoromethyl, difluoromethyl, methoxy, ethoxy, isopropoxy, trifluoromethoxy, difluoromethoxy or trifluoroethoxy;
R₆ is selected from C₁-C₄ alkyl CO₂R₇, CO₂R₇ or CONR₈R₉;
R₇ is selected from hydrogen, C₁-C₄ alkyl, C₁-C₄ haloalkyl, allyl, 2-methylallyl, 1,1-dimethylallyl, allyl carbinyl, propargyl, alkynyl butyl, cyclopropyl, cyclohexyl, C₁-C₃ alkoxy C₁-C₂ alkyl, C₁-C₄ alkoxycarbonyl C₁-C₂ alkyl, C₁-C₄ alkylcarbonyloxy C₂-C₃ alkyl, and benzyl, furylidene or tetrahydrofurylidene which is unsubstituted or further substituted with 1-3 groups independently selected from the following: halogen, CN, NO₂ or C₁-C₄ alkyl;
R₈ and R₉ are respectively selected from hydrogen, C₁-C₄ alkyl, C₁-C₄ haloalkyl or C₁-C₄ alkoxycarbonyl C₁-C₂ alkyl.

A more further preferred compound in the present invention is: in the formula (I)
^{∗} represents an asymmetric carbon atom with configuration S;
R₁ is selected from CH₃ or NH₂;
R₂ is selected from trifluoromethyl, trichloromethyl or difluoromethyl;
R₃ and R₄ are respectively selected from hydrogen, fluorine, chlorine, bromine or isopropoxy;
R₅ is selected from hydrogen, cyano, methyl, ethyl, n-propyl, isopropyl, n-butyl, isobutyl, tert-butyl, trifluoromethyl, difluoromethyl, phenyl, or phenyl substituted independently with 1-4 following substitutents: halogen, CN, NO₂, methyl, isopropyl, tert-butyl, trifluoromethyl, difluoromethyl, methoxy, ethoxy, isopropoxy, trifluoromethoxy, difluoromethoxy or trifluoroethoxy;
R₆ is selected from CH₂CO₂R₇, CH₂CH₂CO₂R₇, CO₂R₇ or CONR₈R₉;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), benzyl, furylidene or tetrahydrofurylidene which is unsubstituted or further substituted with 1-2 groups independently selected from the following: fluorine, chlorine, bromine, CN, NO₂, methyl, isopropyl or tert-butyl;
R₈ is selected from hydrogen, C₁-C₄ alkyl or C₁-C₄ haloalkyl;
R₉ is selected from hydrogen, C₁-C₄ alkyl or C₁-C₄ alkoxycarbonyl C₁-C₂ alkyl.

A further more preferred compound in the present invention is: in the formula (I)
^{∗} represents an asymmetric carbon atom with configuration S;
R₁ is selected from CH₃ or NH₂;
R₂ is selected from trifluoromethyl or difluoromethyl;
R₃ and R₄ are respectively selected from hydrogen, fluorine, chlorine, bromine or isopropoxy;
R₅ is selected from hydrogen, methyl, ethyl, n-propyl, isopropyl, n-butyl, isobutyl or tert-butyl;
R₆ is selected from CH₂CO₂R₇, CH₂CH₂CO₂R₇, CO₂R₇ or CONR₈R₉;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), furanmethylene or tetrahydrofurylidene, and benzyl which is unsubstituted or further substituted with 1-2 groups independently selected from the following: fluorine, chlorine, bromine, CN, NO₂, methyl, isopropyl or tert-butyl;
R₈ is selected from hydrogen, methyl, ethyl, isopropyl, n-propyl, n-butyl, tert-butyl, trifluoroethyl, difluoroethyl, 1-chloroethyl, 1-chloropropyl or 2-chloropropyl;
R₉ is selected from hydrogen, methyl, ethyl, isopropyl, n-propyl, n-butyl, tert-butyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃) or CH₃CH₂OCOCH(CH₃).

The most preferred compound in the present invention is: in the formula (I)
^{∗} represents an asymmetric carbon atom with configuration S;
R₁ is selected from CH₃ or NH₂;
R₂ is selected from trifluoromethyl or difluoromethyl;
R₃ and R₄ are respectively selected from hydrogen, fluorine, chlorine or isopropoxy;
R₅ is selected from hydrogen, methyl, ethyl, n-propyl, isopropyl, n-butyl, isobutyl or tert-butyl;
R₆ is selected from CH₂CO₂R₇, CH₂CH₂CO₂R₇ or CO₂R₇;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), furanmethylene or tetrahydrofurylidene, and benzyl which is unsubstituted or further substituted with 1-2 groups independently selected from the following: fluorine, chlorine, bromine, CN, NO₂, methyl, isopropyl or tert-butyl.

In the definitions of the compounds of the formula (I) provided above, the terms used in the collection are generally defined as follows:
Halogen: fluorine, chlorine, bromine or iodine. Alkyl: linear or branched alkyl, such as methyl, ethyl, propyl, isopropyl, n-butyl or tert-butyl. Cycloalkyl: substituted or unsubstituted cyclic alkyl, such as cyclopropyl, cyclopentyl or cyclohexyl. Substituents such as methyl and halogen. Haloalkyl: linear or branched alkyl on which hydrogen atoms can be partially or fully replaced by halogen atoms, such as chloromethyl, dichloromethyl, trichloromethyl, fluoromethyl, difluoromethyl and trifluoromethyl. Alkoxy: linear or branched alkyl, bonded to the structure through an oxygen atom. Haloalkoxy: linear or branched alkoxyl, and hydrogen atoms on the alkoxyl can be partially or fully replaced by halogen atoms, such as chloromethoxy, dichloromethoxy, trichloromethoxy, fluoromethoxy, difluoromethoxy, trifluoromethoxy, chlorofluoromethoxy and trifluoroethoxy. Alkylthio: linear or branched alkyl, bonded to the structure through a sulfur atom. Halogenated alkylthio: linear or branched alkylthio, and hydrogen atoms on the alkyls can be partially or fully replaced by halogen atoms, such as chloromethylthio, dichloromethylthio, trichloromethylthio, fluoromethylthio, difluoromethylthio, trifluoromethylthio and chlorofluoromethylthio. Alkenyl: linear or branched alkene, such as vinyl, 1-propenyl, 2-propenyl and different butenyl, pentenyl and hexenyl isomers. The alkenyl also comprises polyenes, such as 1,2-propadienyl and 2,4-hexadienyl. Alkylsulfonyl: linear or branched alkyl, connected to the structure through sulfonyl (-SO₂-), such as methylsulfonyl. Aloalkylsulfonyl: linear or branched alkylsulfonyl, and hydrogen atoms on the alkyl can be partially or fully replaced by halogen atoms. Alkylsulfinyl: linear or branched alkyl, connected to the structure through sulfonyl (-SO-), such as methylsulfinyl. Aloalkylsulfonyl: linear or branched alkylsulfinyl, and hydrogen atoms on the alkyl can be partially or fully replaced by halogen atoms. Alkoxycarbonyl: alkoxyl, connected to the structure through carbonyl, such as CH₃OCO- or CH₃CH₂OCO-. Alkoxyalkyl: alkyl-O-alkyl-, such as CH₃OCH₂-. Alkylcarbonyloxy alkyloxycarbonyl: alkyl-CO-O-alkyl-OCO-, such as CH₃COOCH₂OCO-, CH₃COOCH₂CH₂OCO- or C₂H₅COOCH₂CH₂OCO-. Alkylcarbonyl: alkyl, connected to the structure through carbonyl, such as CH₃CO- or CH₃CH₂CO-. Haloalkylcarbonyl: haloalkyl, connected to the structure through carbonyl, such as CF3CO- or CF2HCO-. Cycloalkylcarbonyl: cycloalkyl, connected to the structure through carbonyl, such as cyclopropanoyl or cyclohexylformyl. Halocycloalkylcarbonyl: halocycloalkyl, connected to the structure through carbonyl, such as 1-chlorocyclopropanoyl. Alkylaminosulfonyl: alkyl-NH-SO₂-, such as CH₃NHSO₂- or C₂H₅NHSO₂-. Dialkylaminosulfonyl: dialkyl-N-SO₂-, such as (CH₃)₂NSO₂- or (C₂H₅)₂NSO₂-. Alkylaminocarbonyl: alkyl-NH-CO-, such as CH₃NHCO- or C₂H₅NHCO-. Dialkylaminocarbonyl: dialkyl-N-CO-, such as (CH₃)₂NCO- or (C₂H₅)₂NCO-. Dialkylaminothiocarbonyl: dialkyl-N-CS-, such as (CH₃)₂NCS- or (C₂H₅)₂NCS-. Alkylthioalkylcarbonyl: alkyl-S-alkyl-CO, such as CH₃SCH₂CO or CH₃SCH₂CH₂CO. Aryl: polyaromatic group, such as phenyl and naphthyl. Heteroaryl is a five-membered or six-membered ring containing one or more N, O and S hetero atoms, such as furyl, pyrazolyl, thiazolyl, pyridyl, pyrimidinyl, pyrazinyl, pyridazinyl, triazinyl, quinolinyl, and the like. The central carbon atom at mark ^{∗} is an asymmetric carbon atom or a chiral carbon atom. Four different substituents on the central carbon atom can be R configuration or S configuration according to the R-S systematic nomenclature adopted by IUPAC.

Part of compounds in the present invention can be illustrated by specific compounds listed in Table 1, but the present invention is not limited to the compounds. In the table, the compound R₁=CH₃, R₂=CF3, and other groups are shown in Table 1.

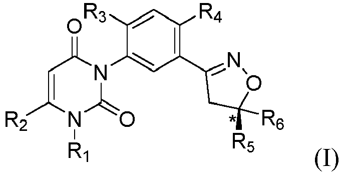

**Table 1**

| No. | R₃ | R₄ | R₅ | R₆ |
|---|---|---|---|---|
| 1 | F | Cl | CH₃ | CO₂H |
| 2 | F | Cl | CH₃ | CO₂CH₃ |
| 3 | F | Cl | CH₃ | CO₂C₂H₅ |
| 4 | F | Cl | CH₃ | CO₂C₃H₇ |
| 5 | F | Cl | CH₃ | CO₂C₄H₉ |
| 6 | F | Cl | CH₃ | CO₂(*cyclo*-C₃H₅) |
| 7 | F | Cl | CH₃ | CO₂(*iso*-C₃H₇) |
| 8 | F | Cl | CH₃ | CO₂(*tert*-C₄H₉) |
| 9 | F | Cl | CH₃ | CO₂CH₂C≡CH |
| 10 | F | Cl | CH₃ | CO₂CH₂CH=CH₂ |
| 11 | F | Cl | CH₃ | CO₂CH₂C(CH₃)=CH₂ |
| 12 | F | Cl | CH₃ | CO₂CH₂CH₂OC₂H₅ |
| 13 | F | Cl | CH₃ | CO₂CH₂CH₂OCH₃ |
| 14 | F | Cl | CH₃ | CO₂CH₂CH₂OCOCH₃ |
| 15 | F | Cl | CH₃ | CO₂CH₂Ph |
| 16 | F | Cl | CH₃ | CO₂CH₂(4-Cl-Ph) |
| 17 | F | Cl | CH₃ | CO₂CH₂(2,6-2F-Ph) |
| 18 | F | Cl | CH₃ | CO₂CH₂(2,6-2Cl-Ph) |
| 19 | F | Cl | CH₃ | CO₂CH₂CF₃ |
| 20 | F | Cl | CH₃ | CO₂CH₂CH(CH₃)₂ |
| 21 | F | Cl | CH₃ | 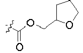 |
| 22 | F | Cl | CH₃ | CH₂CH₂CO₂CH₃ |
| 23 | F | Cl | CH₃ | CH₂CH₂CO₂C₂H₅ |
| 24 | F | Cl | CH₃ | CH₂CO₂CH₃ |
| 25 | F | Cl | CH₃ | CH₂CO₂C₂H₅ |
| 26 | F | Cl | CH₃ | CONH₂ |
| 27 | F | Cl | CH₃ | CONHCH₃ |
| 28 | F | Cl | CH₃ | CONHC₂H₅ |
| 29 | F | Cl | CH₃ | CONHC₃H₇ |
| 30 | F | Cl | CH₃ | CONH(*iso*-C₃H₇) |
| 31 | F | Cl | CH₃ | CONH(*cyclo*-C₃H₅) |
| 32 | F | Cl | CH₃ | CONH(*tert*-C₄H₉) |
| 33 | F | Cl | CH₃ | CON(CH₃)₂ |
| 34 | F | Cl | CH₃ | CON(C₂H₅)₂ |
| 35 | F | Cl | CH₃ | CON(C₃H₇)₂ |
| 36 | F | Cl | CH₃ | CONHCH₂Ph |
| 37 | F | Cl | CH₃ | CONHCH₂CO₂CH₃ |
| 38 | F | Cl | CH₃ | CONHCH(CH₃)CO₂CH₃ |
| 39 | F | Cl | CH₃ | CONHCH₂CH₂CO₂CH₃ |
| 40 | F | Cl | CH₃ | CONHCH₂CO₂C₂H₅ |
| 41 | F | Cl | CH₃ | CONHCH(CH₃)CO₂C₂H₅ |
| 42 | F | Cl | CH₃ | CONHCH₂CH₂CO₂C₂H₅ |
| 43 | F | Cl | H | CO₂H |
| 44 | F | Cl | H | CO₂CH₃ |
| 45 | F | Cl | H | CO₂C₂H₅ |
| 46 | F | Cl | H | CO₂C₃H₇ |
| 47 | F | Cl | H | CO₂C₄H₉ |
| 48 | F | Cl | H | CO₂(*cyclo*-C₃H₅) |
| 49 | F | Cl | H | CO₂(*iso*-C₃H₇) |
| 50 | F | Cl | H | CO₂(*tert*-C₄H₉) |
| 51 | F | Cl | H | CO₂CH₂C≡CH |
| 52 | F | Cl | H | CO₂CH₂CH=CH₂ |
| 53 | F | Cl | H | CO₂CH₂C(CH₃)=CH₂ |
| 54 | F | Cl | H | CO₂CH₂CH₂OC₂H₅ |
| 55 | F | Cl | H | CO₂CH₂CH₂OCH₃ |
| 56 | F | Cl | H | CO₂CH₂CH₂OCOCH₃ |
| 57 | F | Cl | H | CO₂CH₂Ph |
| 58 | F | Cl | H | CO₂CH₂(4-Cl-Ph) |
| 59 | F | Cl | H | CO₂CH₂(2,6-2F-Ph) |
| 60 | F | Cl | H | CO₂CH₂(2,6-2Cl-Ph) |
| 61 | F | Cl | H | CO₂CH₂CF₃ |
| 62 | F | Cl | H | CO₂CH₂CH(CH₃)₂ |
| 63 | F | Cl | H | 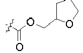 |
| 64 | F | Cl | H | CH₂CH₂CO₂CH₃ |
| 65 | F | Cl | H | CH₂CH₂CO₂C₂H₅ |
| 66 | F | Cl | H | CH₂CO₂CH₃ |
| 67 | F | Cl | H | CH₂CO₂C₂H₅ |
| 68 | F | Cl | H | CONH₂ |
| 69 | F | Cl | H | CONHCH₃ |
| 70 | F | Cl | H | CONHC₂H₅ |
| 71 | F | Cl | H | CONHC₃H₇ |
| 72 | F | Cl | H | CONH(*iso*-C₃H₇) |
| 73 | F | Cl | H | CONH(*cyclo*-C₃H₅) |
| 74 | F | Cl | H | CONH(*tert*-C₄H₉) |
| 75 | F | Cl | H | CON(CH₃)₂ |
| 76 | F | Cl | H | CON(C₂H₅)₂ |
| 77 | F | Cl | H | CON(C₃H₇)₂ |
| 78 | F | Cl | H | CONHCH₂Ph |
| 79 | F | Cl | H | CONHCH₂CO₂CH₃ |
| 80 | F | Cl | H | CONHCH(CH₃)CO₂CH₃ |
| 81 | F | Cl | H | CONHCH₂CH₂CO₂CH₃ |
| 82 | F | Cl | H | CONHCH₂CO₂C₂H₅ |
| 83 | F | Cl | H | CONHCH(CH₃)CO₂C₂H₅ |
| 84 | F | Cl | H | CONHCH₂CH₂CO₂C₂H₅ |
| 85 | H | Cl | CH₃ | CO₂H |
| 86 | H | Cl | CH₃ | CO₂CH₃ |
| 87 | H | Cl | CH₃ | CO₂C₂H₅ |
| 88 | H | Cl | CH₃ | CO₂C₃H₇ |
| 89 | H | Cl | CH₃ | CO₂C₄H₉ |
| 90 | H | Cl | CH₃ | CO₂(*cyclo*-C₃H₅) |
| 91 | H | Cl | CH₃ | CO₂(*iso*-C₃H₇) |
| 92 | H | Cl | CH₃ | CO₂(*tert*-C₄H₉) |
| 93 | H | Cl | CH₃ | CO₂CH₂C≡CH |
| 94 | H | Cl | CH₃ | CO₂CH₂CH=CH₂ |
| 95 | H | Cl | CH₃ | CO₂CH₂C(CH₃)=CH₂ |
| 96 | H | Cl | CH₃ | CO₂CH₂CH₂OC₂H₅ |
| 97 | H | Cl | CH₃ | CO₂CH₂CH₂OCH₃ |
| 98 | H | Cl | CH₃ | CO₂CH₂CH₂OCOCH₃ |
| 99 | H | Cl | CH₃ | CO₂CH₂Ph |
| 100 | H | Cl | CH₃ | CO₂CH₂(4-Cl-Ph) |
| 101 | H | Cl | CH₃ | CO₂CH₂(2,6-2F-Ph) |
| 102 | H | Cl | CH₃ | CO₂CH₂(2,6-2Cl-Ph) |
| 103 | H | Cl | CH₃ | CO₂CH₂CF₃ |
| 104 | H | Cl | CH₃ | CO₂CH₂CH(CH₃)₂ |
| 105 | H | Cl | CH₃ | 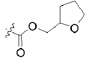 |
| 106 | H | Cl | CH₃ | CH₂CH₂CO₂CH₃ |
| 107 | H | Cl | CH₃ | CH₂CH₂CO₂C₂H₅ |
| 108 | H | Cl | CH₃ | CH₂CO₂CH₃ |
| 109 | H | Cl | CH₃ | CH₂CO₂C₂H₅ |
| 110 | H | Cl | CH₃ | CONH₂ |
| 111 | H | Cl | CH₃ | CONHCH₃ |
| 112 | H | Cl | CH₃ | CONHC₂H₅ |
| 113 | H | Cl | CH₃ | CONHC₃H₇ |
| 114 | H | Cl | CH₃ | CONH(*iso*-C₃H₇) |
| 115 | H | Cl | CH₃ | CONH(*cyclo*-C₃H₅) |
| 116 | H | Cl | CH₃ | CONH(*tert*-C₄H₉) |
| 117 | H | Cl | CH₃ | CON(CH₃)₂ |
| 118 | H | Cl | CH₃ | CON(C₂H₅)₂ |
| 119 | H | Cl | CH₃ | CON(C₃H₇)₂ |
| 120 | H | Cl | CH₃ | CONHCH₂Ph |
| 121 | H | Cl | CH₃ | CONHCH₂CO₂CH₃ |
| 122 | H | Cl | CH₃ | CONHCH(CH₃)CO₂CH₃ |
| 123 | H | Cl | CH₃ | CONHCH₂CH₂CO₂CH₃ |
| 124 | H | Cl | CH₃ | CONHCH₂CO₂C₂H₅ |
| 125 | H | Cl | CH₃ | CONHCH(CH₃)CO₂C₂H₅ |
| 126 | H | Cl | CH₃ | CONHCH₂CH₂CO₂C₂H₅ |
| 127 | H | Cl | H | CO₂H |
| 128 | H | Cl | H | CO₂CH₃ |
| 129 | H | Cl | H | CO₂C₂H₅ |
| 130 | H | Cl | H | CO₂C₃H₇ |
| 131 | H | Cl | H | CO₂C₄H₉ |
| 132 | H | Cl | H | CO₂(*cyclo*-C₃H₅) |
| 133 | H | Cl | H | CO₂(*iso*-C₃H₇) |
| 134 | H | Cl | H | CO₂(*tert*-C₄H₉) |
| 135 | H | Cl | H | CO₂CH₂C≡CH |
| 136 | H | Cl | H | CO₂CH₂CH=CH₂ |
| 137 | H | Cl | H | CO₂CH₂C(CH₃)=CH₂ |
| 138 | H | Cl | H | CO₂CH₂CH₂OC₂H₅ |
| 139 | H | Cl | H | CO₂CH₂CH₂OCH₃ |
| 140 | H | Cl | H | CO₂CH₂CH₂OCOCH₃ |
| 141 | H | Cl | H | CO₂CH₂Ph |
| 142 | H | Cl | H | CO₂CH₂(4-Cl-Ph) |
| 143 | H | Cl | H | CO₂CH₂(2,6-2F-Ph) |
| 144 | H | Cl | H | CO₂CH₂(2,6-2Cl-Ph) |
| 145 | H | Cl | H | CO₂CH₂CF₃ |
| 146 | H | Cl | H | CO₂CH₂CH(CH₃)₂ |
| 147 | H | Cl | H | 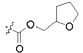 |
| 148 | H | Cl | H | CH₂CH₂CO₂CH₃ |
| 149 | H | Cl | H | CH₂CH₂CO₂C₂H₅ |
| 150 | H | Cl | H | CH₂CO₂CH₃ |
| 151 | H | Cl | H | CH₂CO₂C₂H₅ |
| 152 | H | Cl | H | CONH₂ |
| 153 | H | Cl | H | CONHCH₃ |
| 154 | H | Cl | H | CONHC₂H₅ |
| 155 | H | Cl | H | CONHC₃H₇ |
| 156 | H | Cl | H | CONH(*iso*-C₃H₇) |
| 157 | H | Cl | H | CONH(*cyclo*-C₃H₅) |
| 158 | H | Cl | H | CONH(*tert*-C₄H₉) |
| 159 | H | Cl | H | CON(CH₃)₂ |
| 160 | H | Cl | H | CON(C₂H₅)₂ |
| 161 | H | Cl | H | CON(C₃H₇)₂ |
| 162 | H | Cl | H | CONHCH₂Ph |
| 163 | H | Cl | H | CONHCH₂CO₂CH₃ |
| 164 | H | Cl | H | CONHCH(CH₃)CO₂CH₃ |
| 165 | H | Cl | H | CONHCH₂CH₂CO₂CH₃ |
| 166 | H | Cl | H | CONHCH₂CO₂C₂H₅ |
| 167 | H | Cl | H | CONHCH(CH₃)CO₂C₂H₅ |
| 168 | H | Cl | H | CONHCH₂CH₂CO₂C₂H₅ |

The compound with optical activity in the formula I in the present invention can be prepared from amino compound II with optical activity by the method reported in known literature, such as CN105753853, CN108570041 or WO2016095768.

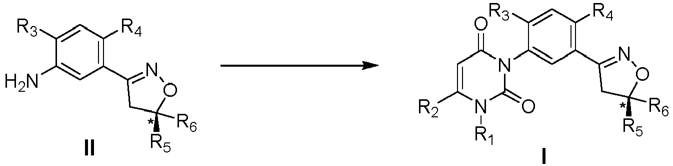

The amino compound II with optical activity can be obtained by resolving the racemic amino compound III using a chiral column. The racemic amino compound III can be prepared by referring to the method in CN105753853.

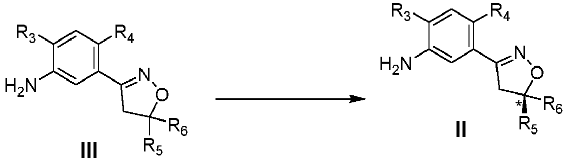

Unless otherwise stated, the definitions of the groups in the reaction formula are the same as above.

An application of the compound of the formula (I) in control for weeds is provided.

The compound of the formula (I) can control various weeds, and can also be used to control resistant weeds.

The compound of the formula (I) can effectively control various broad-leaved weeds, grass weeds, and Cyperaceae weeds, such as *Echinochloa crusgalli, Setaria viridis, Cyperus difformis, Juncellus serotinus,Cyperus esculentus, Digitaria sangunalis, Arthraxon hispidus, Abutilon theophrasti, Zinnia elegans, Amaranthus retrofluxes, Portulaca oleracea, Xanthium sibiricum, Solanum nigrum, Cassia tora, Hibiscus trionum, Glycine soja, Amaranthus palmeri, Amaranthus rudis, Bidens pilosa, Kochia scoparia, Pharbitis purpurea, Eleusine indica, Euphorbia cyathophora, Fagopyrum cymosum, Setaria glauca, Pseudosorghum zollingeri, Conyza canadensis, Alopecurus aequalis, Alopecurus myosuroides, Avena fatua, Poa annua, Avena sterilis, Chenopodium album, Phalaris minor, Raphanus raphanistrum, Stellaria media, Centaurea cyanu, Galium spurium, Lamium maculatum, Tanacetum Parthenium, Papaver rhoeas, Fallopia convolvulus, Veronica persica, Viola tricolor, Festuca arundinacea, Cynodon dactylon, Emilia sonchifolia, Acalypha australis, Commelina communis, Polygonum convolvulus, Sida acuta, Panicum miliaceum, Brachiaria villosa, Celosia argentea, Euphorbia lathyris,* and so on.. The present invention compounds can effectively control weeds even at lower doses.. Moreover, various resistant weeds can be controlled, such as weeds resistant to the herbicides of Acetyl-CoA carboxylase inhibitors, photosystem I, II inhibitors, synthetic auxins, triazines, glyphosate and acetolactate synthase inhibitors.

The compound has certain safety to wheat, corn, rice, soybean, sugar beet and other crops. The compound can be used as a selective herbicide in crop fields in agriculture, and can also be used as a non-selective herbicide in non-cultivated land, fallow land, woodland, orchards and ridges.

The compound of the present invention can be used to control the weeds pre- and post-emergence, and can also be used for soil treatment. Therefore, the present invention also comprises use of the compound of the formula (I) for controlling the weeds.

In addition, the compound of the formula (I) of the present invention is also applicable to drying and/or defoliating plants.

A herbicidal composition uses the compound of the formula (I) as an active ingredient, and the weight percentage of the active ingredient in the composition is 0.1-99%.

An application of the herbicidal composition in control for weeds is provided.

### DESCRIPTION OF THE INVENTION IN DETAIL

The following specific examples are used to further illustrate the present invention, but the present invention is not limited to these examples (unless otherwise specified, the raw materials used are commercially available).

### PREPARATION EXAMPLE

Example 1 The preparation of compound 3 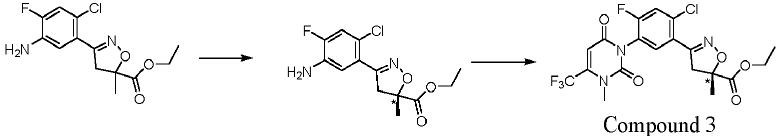
1) Preparation of (S)-3-(5-amino-2-chloro-4-fluorophenyl)-5-methyl-4,5-dihydro-5-isoxazole carboxylic acid ethyl ester
   25g of Amylose tris(3,5-dimethylphenylcarbamate) silica gel was loaded into a chromatographic column; 5 ml of dichloromethane solution containing 1 g of 3-(5-amino-2-chloro-4-fluorophenyl)-5-methyl-4,5-dihydro-5-isoxazole carboxylic acid ethyl ester (prepared according to the method in CN105753853) was adsorbed onto the chromatographic column; Biotage Purification Instrument(Isolera^{™}) was used to separate and collect corresponding components to respectively obtain 0.47 g of optical isomer (R)-3-(5-amino-2-chloro-4-fluorophenyl)-5-methyl-4,5-dihydro-5-isoxazole carboxylic acid ethyl ester (with optical purity of 99.8% and melting point of 105-106°C) and 0.46 g of (S)-3-(5-amino-2-chloro-4-fluorophenyl)-5-methyl-4,5-dihydro-5-isoxazole carboxylic acid ethyl ester (with optical purity of 99.4% and melting point of 104-105°C).
   Optical rotation (Shenguang WZZ-2S/2SS, sodium lamp wavelength: 589.44 nm, solvent: acetonitrile): S isomer [α]^{26°C} _{589.44 nm}=150.65°; R isomer [α]^{26°c} _{589.44 nm}=-111.66°.
2) Preparation of compound 3

0.45 g (1.5 mmol) of (S)-3-(5-amino-2-chloro-4-fluorophenyl)-5-methyl-4,5-dihydro-5-isoxazole carboxylic acid ethyl ester and 0.46 g (1.8 mmol) of ethyl (Z)-3-(3,3-dimethylureido)-4,4,4-trifluorobut-2-enoate (prepared according to the method in CN108570041) were added to a reaction flask containing 20 ml of acetic acid in sequence, the mixture was then heated to reflux, and maintained at the temperature for 6 h; the mixture was concentrated under reduced pressure, followed by adjusting to a pH of 7 using sodium bicarbonate solution, extracted with ethyl acetate, and dried over anhydrous magnesium sulfate and concentrated under reduced pressure, then 0.45 g (3.24 mmol) of potassium carbonate and 50 ml of N,N-dimethylformamide were added in sequence to the residue, the mixture was cooled to 0°C, 0.51 g (3.6 mmol) of iodomethane was added dropwise to the mixture, and then the mixture was raised to room temperature and stirred for 6 h. After the TLC monitored that the reaction was finished, the reactants were poured into water and extracted with ethyl acetate, the organic phase was washed with a saturated brine solution, dried with anhydrous magnesium sulfate, and concentrated under reduced pressure. The residue was purified through silica column (ethyl acetate: petroleum ether=1:5, as an eluent) to obtain compound 3: 0.48 g of oil.

Optical rotation of compound 3 (Shenguang WZZ-2S/2SS, sodium lamp wavelength: 589.44 nm, solvent: acetonitrile): [α]^{20°C} _{589.44 nm}=68.06°.

¹H-NMR (300 MHz, CDCl₃): δ 7.68 (d, *J* = 9.0 Hz, 1H, Ph-H), 7.35 (d, *J* = 9.0 Hz, 1H, Ph-H), 6.36 (s, 1H, Ar-H), 4.26 (q, *J* = 7.1 Hz, 2H, OCH₂), 4.00 (d, *J* = 17.4 Hz, 1H, -CH₂-), 3.56 (s, 3H, N-CH₃), 3.39 (d, *J* = 17.4 Hz, 1H, -CH₂-), 1.71 (s, 3H, CH₃), 1.32 (t, *J* = 7.1Hz, 3H, CH₃).

### Test of Biological ActivityExample 2 Bioassay of Herbicidal Activity in Greenhouse

The test method of the herbicidal activity of the compound in the present invention is as follows:
Quantitative grass weed (*Echinochloa crusgalli* and *Setaria viridis*) and broad-leaved weed (*Abutilon theophrasti* and *Zinnia elegans*) seeds were sown in paper cups having a diameter of 7 cm and containing nutrient soil, after sowing, the seeds were covered with 1 cm of soil, the soil was pressed and watered, and then the seeds were cultivated in a greenhouse according to a conventional method. The grass weeds grew to 2-3 leaf stages, the broad-leaved weeds grew to 2-4 leaf stages, stem and leaves were sprayed. Before emergence, the soil was sprayed within 24 hours after sowing. According to the design dose of the test, spray treatment was carried out on a track-type crop sprayer (designed and produced by British Engineer Research Ltd.) (spray pressure is 1.95 kg/cm², spray volume is 500 L/hm² and track speed is 1.48 km/h). The test was repeated for three times. The test material was treated and then placed in an operation hall. The medicinal liquid was naturally dried in the shade, and then was placed in a greenhouse and managed according to the conventional method. The response of the weeds to the test compound was observed and recorded. After treatment, the control effects of the test compound on the weeds were visually inspected regularly.

The grading standards of the control effects: 0 represents no control effect and 100% represents that the weeds are completely killed or greatly controlled.

It is found through the herbicidal activity test that the post-emergence treatment of the compound 3 at a dose of 4-8 g a.i./hm² has high herbicidal activity on *Abutilon theophrasti, Zinnia elegans, Echinochloa crusgalli, Setaria viridi,* and the activity is significantly better than that of the compound 6 in CN105753853 (Table 2-Table 3).

The specific structural formula of the compound 6 in CN105753853 is as follows:

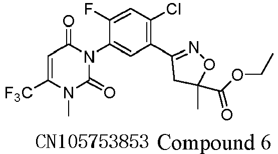

**Table 2 Control Test Results of Grass Weed**

| | Dose g a.i./hm² | *Echinochloa crusgalli* | *Setaria viridis* |
|---|---|---|---|
| 3 | 4 | 90 | 75 |
| | 8 | 98 | 85 |
| CN105753853 Compound 6 | 4 | 45 | 25 |
| | 8 | 50 | 55 |

**Table 3 Control Test Results of Broad-Leaved Weed**

| | Dose g a.i./hm² | *Zinnia elegans* | *Abutilon theophrasti* |
|---|---|---|---|
| 3 | 4 | 100 | 90 |
| CN105753853 Compound 6 | 4 | 90 | 60 |

## Claims

1. An isoxazoline compound with optical activity, **characterized in that** the structure of the compound is shown in formula (I): 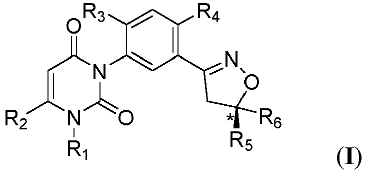 Wherein:
^{∗} represents an asymmetric carbon atom with configuration S;
R₁ is selected from CH₃ or NH₂;
R₂ is selected from C₁-C₄ alkyl or C₁-C₄ haloalkyl;
R₃ and R₄ are respectively selected from hydrogen, halogen, C₁-C₆ alkyl or C₁-C₆ alkoxy;
R₅ is selected from hydrogen, cyano, C₁-C₆ alkyl, C₁-C₆ haloalkyl, C₃-C₆ cycloalkyl, phenyl, or phenyl substituted independently with 1-4 following substitutents: halogen, CN, NO₂, C₁-C₆ alkyl, C₁-C₆ haloalkyl, C₁-C₆ alkoxy, C₁-C₆ haloalkoxy, C₁-C₆ alkylthio or C₁-C₆ alkylsulfonyl;
R₆ is selected from C₁-C₆ alkyl CO₂R₇, CO₂R₇ or CONR₈R₉;
R₇ is selected from hydrogen, C₁-C₆ alkyl, C₁-C₆ haloalkyl, C₃-C₆ cycloalkyl, C₃-C₆ alkenyl, C₃-C₆ alkynyl, C₁-C₆ alkoxy C₁-C₆ alkyl, C₁-C₆ alkoxycarbonyl C₁-C₄ alkyl, C₁-C₆ alkylcarbonyloxy C₂-C₄ alkyl, and benzyl, furylidene, thiazomethylene, tetrahydrofuranmethylene or pyridinemethylene which is unsubstituted or further substituted with 1-4 groups independently selected from the following: halogen, CN, NO₂, C₁-C₈ alkyl, C₁-C₈ haloalkyl, C₁-C₈ alkoxy, C₁-C₈ haloalkoxy, C₁-C₈ alkoxycarbonyl, C₁-C₈ alkylthio or C₁-C₈ alkylsulfonyl;
R₈ and R₉ are respectively selected from hydrogen, C₁-C₆ alkyl, C₁-C₆ haloalkyl or C₁-C₄ alkoxycarbonyl C₁-C₄ alkyl.

2. The compound according to claim 1, **characterized in that** in the formula (I):
^{∗} represents an asymmetric carbon atom with configuration S;
R₁ is selected from CH₃ or NH₂;
R₂ is selected from methyl, isopropyl, tert-butyl, trifluoromethyl, trichloromethyl, difluoromethyl or heptafluoroisopropyl;
R₃ and R₄ are respectively selected from hydrogen, fluorine, chlorine, bromine, methyl, ethyl, isopropyl, tert-butyl, methoxy, ethoxy or isopropoxy;
R₅ is selected from hydrogen, cyano, C₁-C₄ alkyl, C₁-C₄ haloalkyl, C₃-C₆ cycloalkyl, phenyl, or phenyl substituted independently with 1-4 following substitutents: halogen, CN, NO₂, C₁-C₄ alkyl, C₁-C₄ haloalkyl, C₁-C₄ alkoxy, C₁-C₄ haloalkoxy, C₁-C₄ alkylthio or C₁-C₄ alkylsulfonyl;
R₆ is selected from C₁-C₄ alkyl CO₂R₇, CO₂R₇ or CONR₈R₉;
R₇ is selected from hydrogen, C₁-C₆ alkyl, C₁-C₆ haloalkyl, C₃-C₆ cycloalkyl, C₃-C₆ alkenyl, C₃-C₆ alkynyl, C₁-C₄ alkoxy C₁-C₄ alkyl, C₁-C₄ alkoxycarbonyl C₁-C₄ alkyl, C₁-C₄ alkylcarbonyloxy C₂-C₄ alkyl, and benzyl, furylmethylene or tetrahydrofurylidene which is unsubstituted or further substituted with 1-4 groups independently selected from the following: halogen, CN, NO₂, C₁-C₆ alkyl or C₁-C₆ haloalkyl;
R₈ and R₉ are respectively selected from hydrogen, C₁-C₄ alkyl, C₁-C₄ haloalkyl or C₁-C₄ alkoxycarbonyl C₁-C₄ alkyl.

3. The compound according to claim 2, **characterized in that** in the formula (I):
^{∗} represents an asymmetric carbon atom with configuration S;
R₁ is selected from CH₃ or NH₂;
R₂ is selected from methyl, trifluoromethyl, trichloromethyl or difluoromethyl;
R₃ and R₄ are respectively selected from hydrogen, fluorine, chlorine, bromine, methyl, methoxy, ethoxy or isopropoxy;
R₅ is selected from hydrogen, cyano, C₁-C₄ alkyl, C₁-C₄ haloalkyl, C₃-C₆ cycloalkyl, phenyl, or phenyl substituted independently with 1-4 following substitutents: halogen, CN, NO₂, C₁-C₄ alkyl, C₁-C₄ haloalkyl, C₁-C₄ alkoxy or C₁-C₄ haloalkoxy;
R₆ is selected from C₁-C₄ alkyl CO₂R₇, CO₂R₇ or CONR₈R₉;
R₇ is selected from hydrogen, C₁-C₄ alkyl, C₁-C₄ haloalkyl, C₃-C₆ cycloalkyl, C₃-C₆ alkenyl, C₃-C₆ alkynyl, C₁-C₄ alkoxy C₁-C₂ alkyl, C₁-C₄ alkoxycarbonyl C₁-C₂ alkyl, C₁-C₄ alkylcarbonyloxy C₂-C₄ alkyl, and benzyl, furylmethylene or tetrahydrofurylidene which is unsubstituted or further substituted with 1-3 groups independently selected from the following: halogen, CN, NO₂, C₁-C₄ alkyl or C₁-C₄ haloalkyl;
R₈ and R₉ are respectively selected from hydrogen, C₁-C₄ alkyl, C₁-C₄ haloalkyl or C₁-C₄ alkoxycarbonyl C₁-C₂ alkyl.

4. The compound according to claim 3, **characterized in that** in the formula (I):
^{∗} represents an asymmetric carbon atom with configuration S;
R₁ is selected from CH₃ or NH₂;
R₂ is selected from trifluoromethyl, trichloromethyl or difluoromethyl;
R₃ and R₄ are respectively selected from hydrogen, fluorine, chlorine, bromine or isopropoxy;
R₅ is selected from hydrogen, cyano, methyl, ethyl, n-propyl, isopropyl, n-butyl, isobutyl, tert-butyl, trifluoromethyl, difluoromethyl, phenyl, or phenyl substituted independently with 1-4 following substitutents: halogen, CN, NO₂, methyl, isopropyl, tert-butyl, trifluoromethyl, difluoromethyl, methoxy, ethoxy, isopropoxy, trifluoromethoxy, difluoromethoxy or trifluoroethoxy;
R₆ is selected from C₁-C₄ alkyl CO₂R₇, CO₂R₇ or CONR₈R₉;
R₇ is selected from hydrogen, C₁-C₄ alkyl, C₁-C₄ haloalkyl, allyl, 2-methylallyl, 1,1-dimethylallyl, allyl carbinyl, propargyl, alkynyl butyl, cyclopropyl, cyclohexyl, C₁-C₃ alkoxy C₁-C₂ alkyl, C₁-C₄ alkoxycarbonyl C₁-C₂ alkyl, C₁-C₄ alkylcarbonyloxy C₂-C₃ alkyl, and benzyl, furylidene or tetrahydrofurylidene which is unsubstituted or further substituted with 1-3 groups independently selected from the following: halogen, CN, NO₂ or C₁-C₄ alkyl;
R₈ and R₉ are respectively selected from hydrogen, C₁-C₄ alkyl, C₁-C₄ haloalkyl or C₁-C₄ alkoxycarbonyl C₁-C₂ alkyl.

5. The compound according to claim 4, **characterized in that** in the formula (I):
^{∗} represents an asymmetric carbon atom with configuration S;
R₁ is selected from CH₃ or NH₂;
R₂ is selected from trifluoromethyl, trichloromethyl or difluoromethyl;
R₃ and R₄ are respectively selected from hydrogen, fluorine, chlorine, bromine or isopropoxy;
R₅ is selected from hydrogen, cyano, methyl, ethyl, n-propyl, isopropyl, n-butyl, isobutyl, tert-butyl, trifluoromethyl, difluoromethyl, phenyl, or phenyl substituted independently with 1-4 following substitutents: halogen, CN, NO₂, methyl, isopropyl, tert-butyl, trifluoromethyl, difluoromethyl, methoxy, ethoxy, isopropoxy, trifluoromethoxy, difluoromethoxy or trifluoroethoxy;
R₆ is selected from CH₂CO₂R₇, CH₂CH₂CO₂R₇, CO₂R₇ or CONR₈R₉;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), benzyl, furylidene or tetrahydrofurylidene which is unsubstituted or further substituted with 1-2 groups independently selected from the following: fluorine, chlorine, bromine, CN, NO₂, methyl, isopropyl or tert-butyl;
R₈ is selected from hydrogen, C₁-C₄ alkyl or C₁-C₄ haloalkyl;
R₉ is selected from hydrogen, C₁-C₄ alkyl or C₁-C₄ alkoxycarbonyl C₁-C₂ alkyl.

6. The compound according to claim 5, **characterized in that** in the formula (I):
^{∗} represents an asymmetric carbon atom with configuration S;
R₁ is selected from CH₃ or NH₂;
R₂ is selected from trifluoromethyl or difluoromethyl;
R₃ and R₄ are respectively selected from hydrogen, fluorine, chlorine, bromine or isopropoxy;
R₅ is selected from hydrogen, methyl, ethyl, n-propyl, isopropyl, n-butyl, isobutyl or tert-butyl;
R₆ is selected from CH₂CO₂R₇, CH₂CH₂CO₂R₇, CO₂R₇ or CONR₈R₉;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), furanmethylene or tetrahydrofurylidene, and benzyl which is unsubstituted or further substituted with 1-2 groups independently selected from the following: fluorine, chlorine, bromine, CN, NO₂, methyl, isopropyl or tert-butyl;
R₈ is selected from hydrogen, methyl, ethyl, isopropyl, n-propyl, n-butyl, tert-butyl, trifluoroethyl, difluoroethyl, 1-chloroethyl, 1-chloropropyl or 2-chloropropyl;
R₉ is selected from hydrogen, methyl, ethyl, isopropyl, n-propyl, n-butyl, tert-butyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃) or CH₃CH₂OCOCH(CH₃).

7. The compound according to claim 6, **characterized in that** in the formula (I):
^{∗} represents an asymmetric carbon atom with configuration S;
R₁ is selected from CH₃ or NH₂;
R₂ is selected from trifluoromethyl or difluoromethyl;
R₃ and R₄ are respectively selected from hydrogen, fluorine, chlorine or isopropoxy;
R₅ is selected from hydrogen, methyl, ethyl, n-propyl, isopropyl, n-butyl, isobutyl or tert-butyl;
R₆ is selected from CH₂CO₂R₇, CH₂CH₂CO₂R₇ or CO₂R₇;
R₇ is selected from hydrogen, methyl, ethyl, n-propyl, n-butyl, isopropyl, isobutyl, tert-butyl, trifluoroethyl, trifluoromethyl, difluoroethyl, heptafluoroisopropyl, allyl, propargyl, 2-methylallyl, 1,1-dimethylallyl, allyl carbinyl, propargyl, alkynyl butyl, cyclopropyl, cyclohexyl, methoxyethyl, ethoxyethyl, isopropoxyethyl, CH₃OCOCH₂, CH₃CH₂OCOCH₂, (CH₃)₂CHOCOCH₂, CH₃OCOCH₂CH₂, CH₃CH₂OCOCH₂CH₂, (CH₃)₂CHOCOCH₂CH₂, CH₃OCOCH(CH₃), CH₃CH₂OCOCH(CH₃), furanmethylene or tetrahydrofurylidene, and benzyl which is unsubstituted or further substituted with 1-2 groups independently selected from the following: fluorine, chlorine, bromine, CN, NO₂, methyl, isopropyl or tert-butyl.

8. A method of controlling weeds which comprises applying the compound having general formula (I) according to claim 1..

9. A herbicidal composition, **characterized in that** the composition uses the compound of the formula (I) of claim 1 as an active ingredient, wherein the weight percentage of the active ingredient in the composition is 0.1-99%.

10. A method of controlling weeds which comprises applying a herbicidal composition according to claim 9.
